(19) Europäisches Patentamt
European Patent Office
Office européen des brevets

(11) Numéro de publication: 0 112 191
B1

(12) # FASCICULE DE BREVET EUROPÉEN

(45) Date de publication du fascicule du brevet: **04.02.87**

(51) Int. Cl.⁴: **C 07 D 401/04,** A 61 K 31/445

(21) Numéro de dépôt: **83401915.0**

(22) Date de dépôt: **29.09.83**

(54) **Nouveaux dérivés du 4-(1H-indol-3-yl) alpha-méthylpipéridine 1-éthanol, leurs sels, le procédé de préparation, l'application à titre de médicaments et les compositions les renfermant.**

(30) Priorité: **05.10.82 FR 8216669**

(43) Date de publication de la demande:
**27.06.84 Bulletin 84/26**

(45) Mention de la délivrance du brevet:
**04.02.87 Bulletin 87/6**

(84) Etats contractants désignés:
**AT BE CH DE FR GB IT LI LU NL SE**

(56) Documents cité:
**EP-A-0 004 494**
**EP-A-0 022 705**
**FR-A-2 227 873**

**Le dossier contient des informations techniques présentées postérieurement au dépôt de la demande et ne figurant pas dans le présent fascicule.**

(73) Titulaire: **ROUSSEL- UCLAF, 35, boulevard des Invalides, F-75007 Paris (FR)**

(72) Inventeur: **Clemence, François, 2, rue Turgot, F-75009 Paris (FR)**
Inventeur: **Brown, Neil Leslie, 12, rue Jaucourt, F-75012 Paris (FR)**

(74) Mandataire: **Tonnellier, Marie- José, Département des Brevets ROUSSEL UCLAF B.P. no 9, F-93230 Romainville (FR)**

EP 0 112 191 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention concerne de nouveaux dérivés du 4-(1H-indol-3 yl) α-methyl piperidine-1-ethanol, ainsi que leurs sels, le procédé de préparation, l'application à titre de médicaments de ces nouveaux dérivés et les compositions les renfermant.

L'invention a pour objet de nouveaux dérivés du 4-(1H-indol-3-yl) α-méthyl pipéridine-1-éthanol, ainsi que leurs sels d'addition avec les acides minéraux ou organiques, caractérisés en ce qu'ils répondent à la formule générale I:

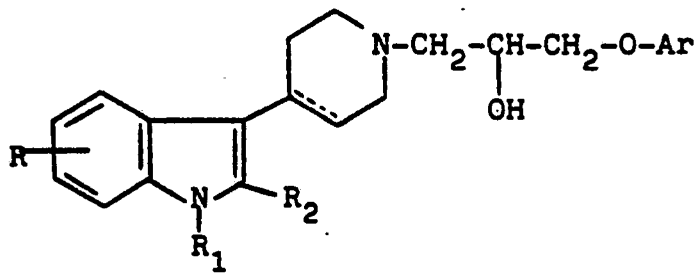

(I)

dans laquelle R représente un atome d'hydrogène ou d'halogène, ou un radical alkyle ou alkoxy renfermant de 1 à 5 atomes de carbone, nitro, amino, trifluorométhyle ou méthylthio, $R_1$ représente un atome d'hydrogène ou un radical alkyle renfermant de 1 à 5 atomes de carbone, $R_2$ représente un atome d'hydrogène, un radical alkyle renfermant de 1 à 5 atomes de carbone ou un radical hydroxy, Ar représente un radical aryle ou hétéroaryle renfermant de 4 à 14 atomes de carbone éventuellement substitué par un ou plusieurs atomes de chlore, brome ou fluor, radicaux hydroxy, hydroxyméthyle, hydroxybutyle, méthoxy, éthoxy, propoxy, méthoxy-éthyle, carbamoyle, propényle, propényloxy, acétamido butyrylamino, acétyle, nitro, amino, méthyle, éthyle, propyle, méthylsulfonamido, cyano, méthoxy-carbonyle, cyclohéxyle, propynyle, éthynyle, propynyloxy ou trifluorométhyle et le pointillé représente la présence éventuelle d'une liaison carbone-carbone.

Dans la formule générale 1 et dans ce qui suit, le terme halogène désigne de préférence un atome de chlore ou de brome; le terme radical alkyle renfermant de 1 à 5 atomes de carbone désigne, de préférence, un radical méthyle, éthyle, n-propyle ou isopropyle; le terme radical alkoxy renfermant de 1 à 5 atomes de carbone designe, de préférence, un radical méthoxy, éthoxy ou propoxy; le terme aryle ou hétéroaryle renfermant de 4 à 14 atomes de carbone désigne de préférence un radical phényle, naphthyle, 1H-indol 4-yl, pyridyl, thienyl, thiazolyl ou thiadiazolyl; l'aryle ou l'hétéroaryle peut comporter un ou plusieurs substituants.

Les sels d'addition avec les acides minéraux ou organiques peuvent être, par exemple, les sels formés avec les acides chlorhydrique, bromhydrique, nitrique, sulfurique, phosphorique acétique, formique, propionique, benzoïque, maléique, fumarique succinique, tartrique, citrique, oxalique, glyoxylique, aspartique, alcane sulfoniques tels que les acides méthane et éthane sulfoniques, arylsulfoniques, tels que les acides benzène et paratoluène sulfoniques et arylcarboxyliques.

Parmi les produits objet de l'invention, on peut citer notamment les dérivés répondant à la formule (1) ci-dessus, ainsi que leurs sels d'addition avec les acides minéraux ou organiques, caractérisés en ce que, dans ladite formule I, $R_2$ représente un atome d'hydrogène ou un radical alkyle renfermant de 1 à 5 atomes de carbone.

Parmi ceux-ci, on peut citer particulièrement les produits caractérisés en ce que, dans la formule I, le pointillé représente un liaison carbone-carbone.

Parmi ces derniers, on peut citer plus particulièrement ceux caractérisés en ce que, dans la formule I, Ar représente un radical phényl éventuellement substitué et ceux caractérisés en ce que $R_1$ et $R_2$ représentent un atome d'hydrogène.

Parmi les produits de formule I, on peut citer tout particulièrement:

le 4-(6-méthoxy 1H-indol-3-yl) α-//2-(prop-2-ényloxy)phénoxy/ méthyl/pipéridine-1-éthanol et le 4-(1H-indol-3-yl) α-//2-prop-2-ényloxy)phényloxy/méthyl/1,2,3,6-tétrahydro pyridin-1-éthanol, ainsi que leurs sels d'addition avec les acides minéraux organiques.

L'invention a également pour objet un procédé de préparation des dérivés, tels que définis par la formule (I) ci-dessus ainsi que de leurs sels, caractérisé en ce que l'on fait réagir un dérivé de l'indole de formule (II):

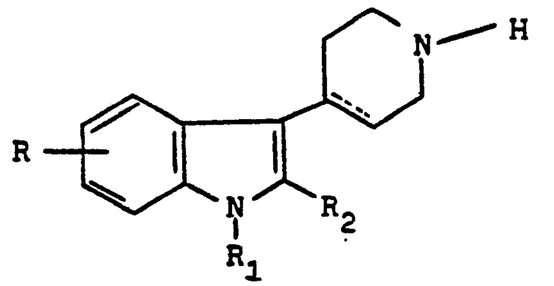

(II)

dans laquelle R, $R_1$ et $R_2$ ont la signification déjà indiquée, avec un époxyde de formule (III):

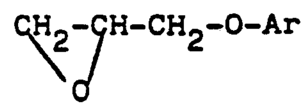

(III)

dans laquelle Ar a la signification déjà indiquée, pour obtenir un produit de formule (I) que l'on isole, et, si désiré, salifie, ou que l'on soumet, si nécessaire, ou si désiré, à une ou plusieurs des réactions suivantes dans un ordre approprié:

- déprotection du ou des groupements hydroxy protégés,
- réduction du ou des groupements nitro en groupements amino,
- acylation du ou des groupements amino,
- salification par un acide minéral ou organique.

Dans des conditions préférentielles de mise en oeuvre de l'invention:

- la réaction de l'indole de formule (II) avec l'époxyde de formule (III) est réalisée dans un mélange méthanol-solvant benzénique au reflux,
- lorsque le groupement Ar est substitué par un ou plusieurs groupements hydroxy, ceux-ci sont protégés sous forme de dérivés benzylés,
- la déprotection du ou des groupements hydroxy est effectuée par réduction à l'hydrogène en présence d'un catalyseur tel que le palladium, dans le cas où le produit de formule I ne comporte pas un cycle tétrahydropyridinyl,
- la réduction du ou des groupements nitro est effectuée par l'hydrogène en présence d'un catalyseur tel que le palladium, dans le cas où le produit de formule I ne comporte pas un cycle tétrahydropyridimyl, cas dans lequel on peut utiliser le système zinc-acide acétique,
- l'acylation du ou des groupements amino est effectuée à l'aide d'un dérivé fonctionnel d'un acyle tel qu'un chlorure ou un anhydride.

Les dérivés de formule (I) présentent un caractère basique. On peut avantageusement préparer les sels d'addition des dérivés de formule (I) en faisant réagir en proportions sensiblement stoechiométriques, un acide minéral ou organique avec ledit dérivé de formule (I). Les sels peuvent être préparés sans isoler les bases correspondantes.

Les dérivés objet de la présente invention possèdent de très intéressantes propriétés pharmacologiques; ils sont doués notamment de remarquables propriétés antagonistes des ions $Ca^{2+}$ et antihypertensives. Certains dérivés possèdent en outre des propriétés α- et ou β -bloquantes.

Ils sont également doués de propriétés anti-arythmiques.

Ces propriétés sont illustrées plus loin dans la partie expérimentale.

Ces propriétés justifient l'utilisation des dérivés du 4-(1H-indol-3-yl) α-méthyl pipéridine-1-éthanol de formule I ainsi que de leurs sels pharmaceutiquement acceptables, à titre de médicaments.

La présente demande a ainsi également pour objet l'application à titre de médicaments, des dérivés du 4-(1H-indol-3-yl) α-méthyl pipéridine-1-éthanol tels que définis par la formule (I) ainsi que de leurs sels d'addition avec les acides pharmaceutiquement acceptables.

Parmi les médicaments selon l'invention, on retient de préférence, les médicaments caractérisés en ce qu'ils sont constitués par les nouveaux dérivés du 4-(1H-indol-3-yl) α-méthyl pipéridine-1-éthanol)répondant à la formule (I) dans laquelle $R_2$ représente un atome d'hydrogène ou un radical alkyle renfermant de 1 à 5 atomes de carbone, ainsi que par leurs sels d'addition avec les acides pharmaceutiquement acceptables.

Parmi ceux-ci, on peut citer particulièrement les produits de formule I dans laquelle le pointillé représente une liaison carbone-carbone.

Parmi ces derniers, on peut citer plus particulièrement les produits de formule I dans laquelle Ar représente un radical phényle éventuellement substitué et les produits de formule I dans laquelle $R_1$ et $R_2$ représente un

atome d'hydrogène.

Parmi les médicaments selon l'invention, on peut citer tout particulièrement:

- le 4-(6-méthoxy 1H-indol-3-yl) α-//2-(prop-2-ényloxy)phénoxy/ méthyl/pipéridine-1-éthanol et le 4-(1H-indol-3-yl) α-//2-(prop-2-ényloxy)phényloxy/méthyl/1,2,3,6-tétrahydro pyridine-1-éthanol, ainsi que leurs sels d'addition avec les acides pharmaceutiquement acceptables.

Les médicaments selon l'invention trouvent leur emploi, par exemple, dans le traitement de l'hypertension artérielle essentielle, de l'hypertension de la cinquantaine, de la ménopause, du diabétique, de l'obèse et du pléthorique, ainsi que dans le traitement de l'hypertension artérielle du sujet âgé ou atteint d'artériosclérose et dans le traitement de l'hypertension d'origine rénale. Ils sont également utilisables dans le traitement de l'insuffisance cardiaque, de l'angor sous toutes ses formes ainsi que dans le traitement des arythmies.

La dose usuelle, variable selon le dérivé utilisé, le sujet traité et l'affection en cause peut être par exemple de 10 mg à 500 mg par jour, par voie orale chez l'homme du dérivé de l'exemple 1 pour le traitement de l'angor.

L'invention a enfin pour objet les compositions pharmaceutiques qui renferment au moins un dérivé précité ou l'un de ses sels d'addition avec les acides pharmaceutiquement acceptables à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple solides ou liquides et se présenter sous les formes pharmaceutiques couramment utilisées en médecine humaine, comme, par exemple, les comprimés, simples ou dragéifiés, les gélules, les granulés, les suppositoires, les préparations injectables, elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les produits de formule (II) sont connus ou peuvent être préparés comme indiqué dans la demande de brevet européen n° 0.022.705. Lorsque l'on veut préparer des produits dont le cycle pipéridyl est saturé, on réduit les produits correspondants de structure tétrahydropyridinyl par l'hydrogène en présence d'un catalyseur tel que le palladium ou par le diborane.

Des produits de formule (II) dont le cycle benzénique est substitué sont décrits par exemple dans Israël J.Chem. 4(4) 155.9(1966), J.Org.Chem. 1979 44(4)578-86, J.Chem.Soc. 1960 526-33, J.Am.Chem.Soc. 77,3839-42 (1955)

Les produits de formule (II) dans laquelle $R_2$ représente un radical hydroxy peuvent être préparés par exemple selon la technique décrite dans le brevet français 2.420.536.

Les produits de formule (III) lorsqu'ils ne sont pas connus, peuvent être préparés par exemple par réaction d'un hydroxyaryle de formule (IV):

Ar-OH (IV)

dans laquelle Ar a la signification déja indiquée avec un 1-halo 2,3-époxy propane, de préférence en présence d'un agent de condensation tel qu'un carbonate ou un bicarbonate alcalin.

Les hydroxy aryles de formule (IV) sont connus ou peuvent être préparés comme indiqué dans le brevet belge n° 640312, la demande de brevet néerlandais n° 66.05.692, les brevets allemands n° 1.236.523 et 2.106.209 le brevet belge n° 669402, les brevets suisses 469002 et 472404, la demande de brevet néerlandaise n° 6612676 ou le brevet sud africain n° 6808345.

Les exemples qui suivent illustrent la présente invention sans toutefois la limiter.

**Exemple 1**: Chlorhydrate de 4-(1H-indol-3-yl) α-//2-(prop-2-ényloxy) phényloxy/méthyl/pipéridine-1-éthanol.

On met en suspension sous atmosphère inerte 5 g de 2-//2-(prop-2-ényloxy)phénoxy/méthyl/oxiranne décrite dans le brevet belge n° 669402 et 5 g de 4-(1H-indol-3-yl)pipéridine dans 100 cm³ de toluène anhydre avec 2 gouttes de méthanol, dissout en chauffant au reflux pendant 5 heures, distille le toluène sous pression réduite, reprend le résidu dans 250 cm³ d'éther éthylique, filtre et ajoute 3,8 cm³ d'acide chlorhydrique. éthanolique 6,4N. On agite encore pendant une heure, essore, empâte à l'éther, sèche en étuve à 60°C sous pression réduite obtient 10,1 g du produit attendu que l'on recristallise dans 60 cm³ d'acétonitrile à chaud, filtre, glace, essore, lave à l'acétonitrile glacé puis à l'éther, sèche à 60°C sous pression réduite et obtient 8 g du chlorhydrate attendu (F 150-155°C).

Analyse: pour $C_{25}H_{31}ClN_2O_3$ = 442,98

Calculé: C % 67,78 H % 7,05 Cl % 8,00 N % 6,33

Trouvé: 67,9 6,9 8,0 6,4

**Exemple 2**: 4-(1H-indol-3-yl) α-/1-(naphtyloxy)méthyl/pipéridine-1

éthanol et son chlorhydrate.

On agite sous atmosphère inerte, 6 g de 4-(1H-indol-3-yl) α-/1-(naphtyloxy)méthyl/pipéridine dans 50 $cm^3$ de benzène et 15 $cm^3$ de méthanol et 66 g de (1-naphthyloxy)méthyl oxiranne décrite dans J.Med.Chem. 19 1255 dans 40 $cm^3$ de benzène porte 20 heures au reflux, ajoute alors 15 $cm^3$ de méthanol et agite encore au reflux pendant 6 heures. On refroidit, concentre sous pression réduite, empâte le produit cristallisé obtenu dans 100 $cm^3$ de méthanol au reflux, glace, essore, sèche à 80°C sous pression réduite et obtient 8 g du produit attendu (F≈ 185°C).

**Formation du chlorhydrate**:

On dissout à chaud la base ci-dessus dans 200 $cm^3$ de chlorure de méthylène et 125 $cm^3$ de méthanol, filtre, ajoute 4 $cm^3$ d'éther chlorhydrique 5N et concentre. On essore, lave et sèche à 110°C sous pression réduite le produit cristallisé et obtient 6,75 g du chlorhydrate attendu (F≈240°C).

**Analyse**: $C_{26}H_{29}ClN_2O_2$ = 436,98

Calculé: C % 71,46 H % 6,68 Cl % 8,11 N % 6,41
Trouvé: 71,2 6,9 8,2 6,4.

**Exemples 3 à 41**

On opère de manière analogue à celle décrite à l'exemple 1 ou 2, en utilisant au départ une 4-(1H-indol-3-yl)pipéridine et un méthyloxiranne appropriés, pour préparer les produits dont les noms et les constantes physiques sont indiqués ci-après:

**Exemple 3**:

Chlorhydrate de 4-(1H-indol-3-yl) α-/(phényloxy)méthyl/ pipéridine-1-éthanol. F + 252-253°C.

**Exemple 4**:

Chlorhydrate de 4-(1H-indol-3-yl) α-/(2-cyanophényloxy)méthyl/ pipéridine-1-éthanol F = 123°C Rf. = 0,35 Silice(acétate d'éthyle-méthanol-triéthylamine 92-5-3).

**Exemple 5**:

Chlorhydrate de 4-(1H-indol-3-yl) α-/(4-nitro phényloxy) méthyl/pipéridine-1-éthanol. F = 172°C.

**Exemple 6**:

Chlorhydrate de 4-(1H-indol-3-yl) α-/(2-méthoxycarbonyl phényloxy)méthyl/pipéridine-1-éthanol. Rf. = 0,27 Silice (acétate d'éthyle-méthanol-triéthylamine 92-5-3).

**Exemple 7**:

Chlorhydrate de 4(1H-indol-3-yl) α-/(2-(prop-2-ényl)phényl oxy) méthyl/pipéridine-1-éthanol. F = 165°C.

**Exemple 8:**

Chlorhydrate de 4-(1H-indol-3-yl) α-/(4-(méthoxyphényloxy) méthyl/pipéridine-1-éthanol. F = 228° C.

**Exemple 9**

Chlorhydrate de 4-(1H-indol-3-yl) α-/(2-méthylphényloxy) méthyl/pipéridine-1-éthanol. F = 240° C (sublimation).

**Exemple 10:**

Chlorhydrate de 4-(1H-indol-3-yl) α-/(2-chlorophényloxy) méthyl/pipéridine-1-éthanol. F = 223° C.

**Exemple 11:**

Chlorhydrate de 4-(1H-indol-3-yl) α-/(2-nitrophényloxy) méthyl/pipéridine-1-éthanol. F = 196° C.

**Exemple 12:**

Chlorhydrate de 4-(1H-indol-3-yl) α-/(2-fluorophényloxy) méthyl/pipéridine-1-éthanol. F = 189° C.

**Exemple 13:**

Chlorhydrate de 4-(1H-indol-3-yl) α-/(2-hydroxy phényloxy)méthyl/pipéridine-1-éthanol. F = 163° C. Ce produit a été préparé en utilisant au départ un 2-hydroxy phényl oxyméthyl oxiranne dont le radical hydroxy est protégé sous forme de dérivé benzylé, la déprotection étant opérée par réduction; à l'aide d'hydrogène sur palladium dans l'acétate d'éthyle.

**Exemple 14:**

Chlorhydrate de 4-(1H-indol-3-yl) α-/(2-méthoxy phényloxy)méthyl/pipéridine-1-éthanol. F = 215° C.

**Exemple 15:**

Chlorhydrate de 4-(1H-indol-3-yl) α-/(2-cyclohexyl phényl oxy)méthyl/pipéridine-1-éthanol. F = 216-8° C

**Exemple 16:**

Chlorhydrate de 4-(1H-indol-3-yl) α-/(2-prop-2-ynyl)oxy phényl oxy)méthyl/pipéridine-1-éthanol. F = 124° C.

**Exemple 17:**

Chlorhydrate de 4-(1H-indol-3-yl) α-/(2-acétyl phényl oxy)méthyl/pipéridine-1-éthanol. F = 200° C.

**Exemple 18:**

Chlorhydrate de 4-(1H-indol-3-yl) α-/(3-méthyl phényl oxy)méthyl/pipéridine-1-éthanol. Rf. = 0,22 Silice (acétate d'éthyle à 1 % de triéthylamine.

**Exemple 19:**

Fumarate de 4-(1H-indol-3-yl) α-/(2-aminophényl oxy)méthyl/pipéridine-1-éthanol. F = 220° C. Ce produit a été préparé par réduction à l'aide d'hydrogène en présence de palladium dans le méthanol, du produit de l'exemple 11, puis salification par l'acide fumarique.

**Exemple 20:**

Chlorhydrate de 4-(1H-indol-3-yl) α-/(2-acétamido phényloxy)méthyl/pipéridine-1-éthanol. F = 243° C.

Ce produit a été préparé au départ du produit de l'exemple 19 (base) par traitement au chlorure d'acétyle dans le benzène, en présence de triéthylamine, puis traitement à la soude dans l'éthanol et enfin salification par l'acide chlorhydrique.

**Exemple 21:**

Chlorhydrate de 4-(1H-indol-3-yl) α-/(4-méthyl phényloxy)méthyl/pipéridine-1-éthanol. F = 216-218° C.

**Exemple 22:**

Chlorhydrate de 4-(1H-indol-3-yl) α-/(3-chloro phényloxy)méthyl/pipéridine-1-éthanol. F = 226° C.

**Exemple 23:**

Chlorhydrate de 4-(1H-indol-3-yl) α-/(3-méthoxy phényloxy)méthyl/pipéridine-1-éthanol. F = 165° C.

**Exemple 24:**

Fumarate acide de 4-(1H-indol-3-yl) α-/(2-(prop-2-enyloxy)phényloxy)méthyl)1,2,3,6-tétrahydropyridine-1-éthanol. F = 176° C.

**Exemple 25:**

Chlorhydrate de 4-(1H-indol-3-yl) α-/(4-trifluoro-méthylphényloxy) méthyl/pipéridine-1-éthanol. F = 205° C.

**Exemple 26:**

Chlorhydrate de 4-(1H-indol-3-yl) α-/(4-(prop-2-en yloxy)phényloxy)méthyl/pipéridine-1-éthanol. F = 214° C.

**Exemple 27:**

Chlorhydrate de 4-(1H-indol-3-yl) α-/(3,4-diméthoxy phényloxy)méthyl/pipéridine-1-éthanol. F = 206° C.

**Exemple 28:**

Chlorhydrate de 4-(1H-indol-3-yl) α-/(3-(prop-2-en yloxy)phényloxy)méthyl/pipéridine-1-éthanol. F = 132° C.

**Exemple 29:**

Chlorhydrate de 4-(1H-indol-3-yl) α-/(3,4-dichloro phényloxy) méthyl/pipéridine-1-éthanol. F = 215° C.

**Exemple 30:**

Chlorhydrate de 4-(1H-indol-3-yl) α-/(3-hydroxy phényloxy)méthyl/pipéridine-1-éthanol. F = 160° C. Ce produit a été préparé comme celui de l'exemple 13 en utilisant un oxiranne protégé par un benzyle, puis en réduisant par l'hydrogène en présence de palladium dans l'acétate d'éthyle.

**Exemple 31:**

Fumarate de 4-(1-méthyl 1H-indol-3-yl) α-/(2-(prop-2-enyloxy) phényloxy)méthyl/pipéridine-1-éthanol. F= 100° C. Rf. 0,25 silice(acétate d'éthyle à 1 % de triéthylamine).

**Exemple 32:**

Fumarate de 4-(5-méthoxy 1H-indol-3-yl) α-/(2-prop-2-enyloxy) phényloxy)méthyl/pipéridine-1-éthanol. Rf. 0,15 silice(acétate d'éthyle à 1 % de triéthylamine).

**Exemple 33:**

Chlorhydrate de 4-(6-méthoxy-1H-indol-3-yl) α-/(2-(prop-2-enyloxy) phényloxy)méthyl/pipéridine-1-éthanol. F = 147-148° C.

**Exemple 34:**

Chlorhydrate de 4-(1H-indol-3-yl) α-/(3-(2-méthoxycarbonyl)thiényloxy)méthyl/pipéridine-1-éthanol. F = 200° C.

**Exemple 35:**

Chlorhydrate de 4-(6-chloro 1H-indol-3-yl) α-/(2-(prop-2-enyloxy)phényloxy)méthyl/pipéridine-1-éthanol.

**Exemple 36:**

Chlorhydrate de 4-(6-méthyl 1H-indol-3-yl) α-/(2-prop-2-enyloxy)phényloxy)méthyl/pipéridine-1-éthanol.

**Exemple 37:**

Chlorhydrate de 4-(6-nitro 1H-indol-3-yl) α-/(2-prop-2-enyloxy)phényloxy) méthyl/pipéridine-1-éthanol.

**Exemple 38:**

Chlorhydrate de 4-(6-méthylthio 1H-indol-3-yl) α-/(2-prop-2-enyloxy)phényloxy)méthyl/pipéridine-1-éthanol.

**Exemple 39:**

Chlorhydrate de 4-(6-trifluorométhyl 1H-indol-3-yl) α-/(2-prop-2-enyloxy)phényloxy)méthyl/pipéridine-1-éthanol.

**Exemple 40:**

Chlorhydrate de 4-(2-méthyl 6-méthoxy 1H-indol-3-yl) α-/(2-prop-2-enyloxy)phényloxy)méthyl/pipéridine-1-éthanol. Rf.: = 0,3 silice(acétate d'éthyle à 1 % de triéthylamine.

**Exemple 41:**

Fumarate acide de 4-(2-hydroxy 1H-indol-3-yl) α-/(2-prop-2-enyloxy)phényloxy)méthyl/pipéridine-1-éthanol. F = 172-174°C.

**Exemple 42:**

On a préparé des comprimés sécables répondant à la formule: - chlorhydrate de 4-(6-méthoxy 1H-indol-3-yl) α-//2-(prop-2-enyloxy) phényloxy)méthyl/pipéridine-1-éthanol........ 20 mg
- excipient q.s. pour un comprimé terminé à........... 100 mg
(Détail de l'excipient: lactose, amidon, talc, stéarate de magnésium).

**Exemple 43:**

On a préparé des comprimés répondant à la formule:
- chlorhydrate de 4-(1H-indol-3-yl) α-//2-(prop-2-ényloxy) phényloxy/méthyl/1,2,3,6-tétrahydropyridine-1-éthanol.... 75 mg
- excipient q.s. pour un comprimé terminé à............ 100 mg
(Détail de l'excipient: lactose, amidon, talc, stéarate de magnésium).

**Etude pharmacologique.**

1) Test d'activité anticalciqué in vitro.
Des artères de rat découpées en spirale sont reliées à des capteurs de tension, et sont maintenues dans des cuves de 2,5 ml de tampon Krebs-bicarbonate de sodium (NaCl: 120,8 mM, KCl: 5,9 mM, $MgCl_2$: 1,2 mM, $NaH_2PO_4$: 1,2 mM, $NaHCO_3$: 15,5 mM, glucose: 12,6 mM) à 37°C gazées avec un mélange $O_2$: 95% - $CO_2$: 5 %.

Les préparations sont dépolarisées par une solution tampon à concentration 100 mM en ions $K^+$ (NaCl: 26,7 mM, KCl; 100 mM, $MgCl_2$: 1,2 mM, $NaH_2PO_4$: 1,2 mM, $NaHCO_3$: 15,5 mM, glucose: 12,6 mM).

On ajoute sous un volume de 25 µl du chlorure de calcium, de manière à obtenir une gamme de concentrations croissantes en ions $Ca^{2+}$ allant de 0,1 à 3,0 mM, on enregistre les contractions des artères et établit ainsi une gamme témoin. On répète l'opération avec la gamme d'ions $Ca^{2+}$ toutes les 15 minutes et la préparation est lavée quatre fois après chaque gamme.

Lorsque l'on obtient une réponse stable, l'on effectue l'opération avec les gammes d'ions CA en presence de différentes concentrations du produit à tester, jusqu'à ce qu'une réponse stable soit obtenue.

Les contractions des artères dépendent de l'entrée des ions $Ca^{2+}$ dans les cellules des muscles lisses et sont provoquées par la dépolarisation du muscle lisse par les ions $K^+$ et par l'action de la noradrénaline libérée au niveau présynaptique. En recommençant l'opération avec des artères dénervées par action de 6-OH dopamine, on supprime l'action propre due à la noradrénaline.

Les résultats sont exprimés en CI 50 (concentration inhibitrice 50) concentration du produit testé qui inhibe

de 50 % la contraction due aux ions K+.

On constate d'après les résultats figurant sur le tableau n° 1 que les produits de la présente demande possèdent une forte activité anticalcique.

**Tableau 1**

| Exemple | $CI_{50}$ en $\mu M$ |
|---|---|
| 1 | 4,0 |
| 2 | 3,5 |
| 3 | 2,5 |
| 4 | 3,5 |
| 5 | 4,5 |
| 6 | 3,0 |
| 7 | 0,32 |
| 8 | 4,2 |
| 9 | 3,8 |
| 10 | 2,0 |
| 11 | 4,2 |
| 12 | 3,2 |
| 13 | 4,5 |
| 14 | 11,0 |
| 15 | 5,0 |
| 16 | 1,6 |
| 17 | 6,0 |
| 18 | 2,0 |
| 21 | 1,8 |
| 22 | 1,8 |
| 23 | 2,7 |
| 24 | 0,7 |
| 25 | 1,5 |
| 26 | 1,5 |
| 28 | 2,4 |
| 29 | 2,4 |
| 31 | 3,0 |
| 32 | 1,9 |

2) Action antiarythmique chez le cobaye

On trachéotomise des cobayes mâles pesant 450-500 g anesthésiés par voie intrapéritonéale à l'aide de 1,25 m/g/kg d'uréthanne et les soumet à une respiration artificielle (40-50 insufflations de 5 ml/minute).

On implante des aiguilles en sous cutané de manière à enregistrer l'électrocardiogramme des cobayes sur le signal en dérivation DII.

On administre les produits à tester per os.

Une heure après l'administration du produit, on perfuse la veine jugulaire des cobayes avec 0,5 ml/mn d'une solution de K-strophanthine à 150 γ/ml et 0 5 ml/mn d,une solution d'adrénaline à 5 γ/ml et on note le temps d'apparition des troubles du rythme cardiaque.

Les résultats sont exprimés en pourcentage de protection à la dose orale de 10 mg/kg de produit à tester, c'est-à-dire en pourcentage d'allongement du temps d'apparition des troubles du rythme cardiaque par rapport aux témoins.

Les résultats figurant sur le tableau n° 2 montrent que les produits de la présente demande sont en général doués de bonnes propriétés antiarythmiques.

**Tableau 2**

| Exemple | Pourcentage de protection |
|---|---|
| 1 | 63 |
| 3 | 55 |
| 4 | 148 |
| 5 | 82 |
| 6 | 37 |
| 7 | 50 |
| 8 | 57 |
| 9 | 48 |
| 10. | 59 |
| 11 | 56 |
| 12 | 90 |
| 13 | 112 |
| 14 | 83 |
| 16 | 71 |
| 17 | 142 |
| 18 | 23 |
| 19 | 165 |
| 21 | 60 |
| 23 | 73 |
| 25 | 35 |
| 26 | 68 |
| 27 | 115 |
| 28 | 103 |
| 29 | 53 |
| 30 | 86 |
| 31 | 49 |
| 32 | 20 |

3) Détermination de l'activité hypotensive.

L'activité hypotensive a été étudiée sur des rats mâles de souche WISTAR pesant 300 g environ et anesthésiés au nembutal (50 mg/kg par voie intraveineuse).

Le produit testé a été administré par voie intraveineuse dans la veine jugulaire.

La pression artérielle carotidienne a été mesurée avant et après administration du produit testé.

Le tableau ci-aprés indique les variations exprimées en pourcentage de la pression artérielle après administration du produit testé par rapport à la pression artérielle témoin initiale.

| Produit de l'exemple | Dose mg/kg | VARIATION % DE LA PRESSION ARTERIELLE | | | |
|---|---|---|---|---|---|
| | | 1 minute après l'adminis-tration | 5 minutes après l'adminis-tration | 10 minutes après l'adminis-tration | 30 minutes après l'adminis-tration |
| 4 | 1 | - 24 | - 21 | - 22 | - 26 |
| 6 | 1 | - 21 | - 13 | - 13 | 0 |
| 8 | 1 | - 10 | - 13 | 0 | - 13 |
| 9 | 1 | - 21 | - 9 | - 25 | - 20 |
| 10 | 1 | - 21 | - 20 | - 12 | - 20 |
| 11 | 0,1 | - 3 | - 7 | - 12 | - 16 |
| 12 | 1 | - 27 | - 14 | - 27 | - 24 |
| 16 | 0,1 | - 3 | - 6 | - 9 | - 20 |
| 17 | 1 | - 17 | - 5 | - 18 | - 6 |
| 23 | 1 | - 7 | - 19 | + 14 | - 17 |
| 24 | 1 | - 10 | - 16 | - 11 | - 17 |
| 25 | 1 | - 5 | - 14 | - 6 | - 14 |
| 28 | 1 | - 5 | - 10 | - 6 | - 9 |

4) Affinité pour les récepteurs α adrenergiques

La technique est inspirée de celle de Mohler et Okada: Science, Vol. 198 p. 849-851 (1977).

On homogénéise dans 90 ml de tampon Tris HCl pH 7,7 cinq cerveaux entiers prélevés sur des rats mâles pesant 150 g en moyenne. Après centrifugation à 30 000 g pendant 15 minutes à 0, + 4°C, le culot est mis en suspension dans 240 ml de tampon Tris HCl 50 mM pH 7,7 et centrifugé à 30 000 g pendant 20 minutes à 0, + 4°C. Le nouveau culot obtenu est mis en suspension dans 480 ml de tampon Krebs Tris HCl pH 7,7/50 mM.

On fait ensuite incuber pendant 30 minutes à 25°C, 2 ml de suspension en présence de 3H prazosine à la concentration 0,12nM i) seule; ii) avec des concentrations croissantes du produit à tester, ou iii) pour déterminer la fixation non spécifique, avec de la phentolamine non radioactive à la concentration $10^{-5}$M.

Les suspensions incubées sont filtrées sur Whatman GF/C et les filtres sont lavés par trois fois 5 ml de tampon Krebs Tris HCl pH 7,7 à 0, + 4°C.

La radioactivité des filtres est mesurée par scintillation liquide.

L'affinité du produit testé pour les récepteurs $\alpha_1$ adrénergiques est donnée relativement à la phentolamine comme produit de référence.

CD = concentration de phentolamine inhibant 50 % de la fixation spécifique de la 3H prazosine;

CX = Concentration du produit à tester inhibant 50 % de la fixation spécifique de la 3H prazosine.

L'affinite relative est donnee par la relation $ARL = 100 \frac{CD}{CX}$

On a obtenu les résultats suivants: PRODUIT DE L'EXEMPLE ARL en %

| PRODUIT DE L'EXEMPLE | ARL en % |
|---|---|
| 1 | 247 |
| 4 | 145 |
| 5 | 77 |
| 6 | 253 |
| 7 | 100 |
| 8 | 222 |
| 9 | 92 |
| 11 | 77 |
| 12 | 233 |
| 13 | 293 |
| 16 | 321 |
| 17 | 159 |
| 20 | 311 |
| 21 | 93 |
| 22 | 70 |

On constate que les produits ci-dessus en particulier possèdent une remarquable affinité pour les récepteurs $\alpha_1$ adrénergiques.

5) Affinité pour les récepteurs $\beta_1$ adrenergiques

La technique est inspirée de celle de Möhler et Okada: Science, Vol. 198 p. 849-851 (1977).

On homogénéise dans 90 ml de sucrose 0,32 M, 10 cortex prélevés sur des cerveaux de rats mâles pesant 150 g en moyenne. Après centrifugation à 1000 g du mélange homogénéisé pendant 20 minutes à 0°C, le surnageant est centrifugé à 30 000 g pendant 15 minutes à 0, + 4°C.

Le culot est mis en suspension dans 120 ml de tampon Tris HCl 50mM pH 7,7 et centrifugé à 30 000 g pendant 15 minutes à 0,+ 4°C. Le nouveau culot obtenu est mis en suspension dans 480 ml de tampon Krebs Tris HCl pH 7,7 50 mM.

On fait ensuite incuber pendant 10 minutes à 37°C, 2 ml de suspension en présence de 3H dihydroalprénolol à la concentration $10^{-9}$ M, i) seule, ii) avec des concentrations croissantes du produit à tester ou iii) pour déterminer la fixation non spécifique avec du propranolol non radioactif à la concentration $10^{-5}$ M.

Les suspensions incubées sont filtrées sur Whatman GF/C et les filtres sont lavés par trois fois 5 ml de tampon Krebs Tris HCl pH 7,7 à 0°C.

La radioactivité des filtres est mesurée par scintillation liquide.

L'affinité du produit testé pour les récepteurs adrénergiques est donnée relativement au propranolol comme produit de référence.

CD = concentration de propranolol inhibant 50 % de la fixation spécifique du 3H dihydroalprénolol.

CX = Concentration du produit à tester inhibant 50 % de la fixation spécifique du 3H dihydroalprénolol.

L'affinité relative est donnée par la relation $ARL = 100 \frac{CD}{CX}$

On a obtenu les résultats suivants :

| Produit de l'exemple | ARL en % |
|---|---|
| 1 | 7,3 |
| 2 | 0,9 |
| 4 | 13,3 |
| 6 | 1,3 |
| 7 | 3,7 |
| 9 | 2,55 |
| 10 | 1,95 |
| 11 | 1,2 |
| 12 | 1,2 |
| 13 | 1,1 |
| 16 | 5,8 |
| 17 | 1,2. |

On constate que les produits ci-dessus en particulier possèdent une notable affinité pour les récepteurs $\beta_1$ adrénergiques.

6) Affinité pour les récepteurs $\beta_2$ adrénergiques:

La technique est inspirée de celle de Höhler et Okada: Science Vol. 198 p. 849-851 (1977).

On homogénéise dans 90 ml de sucrose 0,32M les cervelets prélevés sur des cerveaux de rats mâles pesant 150 g en moyenne. Après centrifugation à 1 000 g du mélange homogénéisé pendant 20 minutes à 0°C, le surnageant est centrifugé à 30 000 g pendant 15 minutes à 0, + 4°C.

Le culot est mis en suspension dans 120 ml de tampon Tris HCl 50 mM pH 7,7 et centrifugé à 30 000 g pendant 15 minutes à 0, + 4°C. Le nouveau culot obtenu est mis en suspension dans 480 ml de tampon Krebs Tris HCl pH 7,7.

On fait ensuite incuber pendant 10 minutes à 37°C, 2 ml de suspension en présence de 3H dihydroalprénobol à la concentration $10^{-9}$ M, i) seule, ii) avec des concentrations croissantes du produit à tester, ou iii) pour déterminer la fixation non spécifique, avec du propranolol non radioactif à la concentration $10^{-5}$M.

Les suspensions incubées sont filtrées sur Whatman GF/C et les filtres sont lavés par trois fois 5 ml de tampon Krebs Tris HCl pH 7,7 à 0°C.

La radioactivité des filtres est mesurée par scintillation liquide.

L'affinité du produit testé pour les récepteurs $\beta_2$ adrénergiques est donnée relativement au propranotol comme produit de référence.

CD = concentration de propranolol inhibant 50 % de la fixation spécifique du 3H dihydroalprénolol.

CX = Concentration du produit à tester inhibant 50 % de la fixation spécifique du 3H dihydroalprénolol.

L'affinité relative est donnée par la relation ARL 100 $\frac{CD}{CX}$

On a obtenu les résultats suivants :

| Produit de l'exemple | ARL en % |
|---|---|
| 1 | 10,5 |
| 4 | 1,1 |
| 6 | 3,5 |
| 7 | 10 |
| 15 | 5,4 |
| 16 | 11 |
| 17 | 3,3 |

On constate que les produits ci-dessus en particulier possèdent une notable affinité pour les récepteurs $R_2$ adrénergiques.

3) <u>Etude de la toxicité aigüe</u>.

On a évalué les doses létales $DL_0$ des composés testés après administration par voie orale chez la souris.

On appelle $DL_0$ la dose maximale ne provoquant aucune mortalité en 8 jours.

<u>Les résultats obtenus sont les suivants:</u>

| Exemple | $DL_0$ |
|---|---|
| 1 | 100 |
| 2 | > 1000 |
| 4 | > 100 |
| 5 | > 200 |
| 6 | 80 |
| 7 | > 200 |
| 8 | > 200 |
| 9 | > 100 |
| 10 | > 100 |
| 11 | 100 |
| 12 | 80 |
| 13 | > 100 |
| 14 | > 100 |
| 15 | > 100 |
| 16 | > 100 |
| 17 | > 100 |
| 18 | > 100 |
| 19 | > 200 |
| 21 | > 100 |
| 22 | 80 |
| 23 | > 200 |
| 24 | > 200 |
| 25 | 100 |
| 26 | > 200 |
| 27 | 80 |
| 28 | > 100 |
| 29 | > 100 |
| 30 | > 100 |

**Revendications** pour les Etats contractants BE CH DE FR CB IT LI LU NL SE

1) Les dérivés du 4-(1H-indol-3-yl) α-méthyl pipéridine-1-éthanol, ainsi que leurs sels d'addition avec les acides minéraux et organiques, caractérisés en ce qu'ils répondent à la formule générale (I).

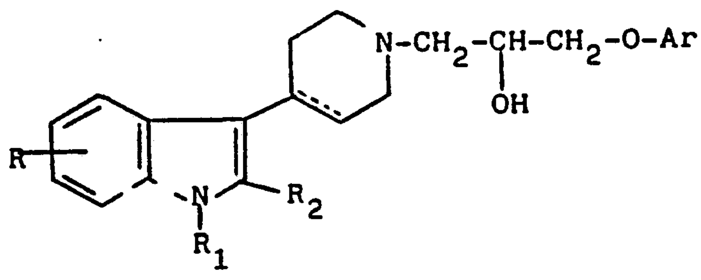

(I)

dans laquelle R represente un atome d'hydrogène ou d'halogène, ou un radical alkyle ou alkoxy renfermant de 1 à 5 atomes de carbone, nitro, amino, trifluorométhyle ou méthylthio, $R_1$ représente un atome d'hydrogène ou un radical alkyle renfermant de 1 à 5 atomes de carbone, $R_2$ représente un atome d'hydrogène, un radical alkyle renfermant de 1 à 5 atomes de carbone ou un radical hydroxy, Ar représente un radical aryle ou hétéroaryle renfermant de 4 à 14 atomes de carbone éventuellement substitué par un ou plusieurs atomes de chlore, brome ou fluor, radicaux hydroxy, hydroxyméthyle, hydroxybutyle, méthoxy, éthoxy, propoxy, méthoxy-éthyle, carbamoyle, propényle, propényloxy, acétamido, butyrylamino, acétyle, nitro, amino, méthyle, éthyle, propyle, méthyl-sulfonamido, cyano, méthoxycarbonyle, cyclohexyle, propynyle, éthynyle, propynyloxy ou trifluorométhyle et le pointillé représente la présence éventuelle d'une liaison carbone-carbone.

2) Les derives du 4-(1H-indol-3-yl) α-méthyl pipéridine-1-éthanol, tels que définis par la formule (I) de la revendication 1, ainsi que leurs sels d'addition avec les acides minéraux ou organiques, caractérisés en ce que $R_2$ représente un atome d'hydrogène ou un radical alkyle renfermant de 1 à 5 atomes de carbone.

3) Les dérivés selon la revendication 1 ou 2, ainsi que leurs sels d'addition avec les acides minéraux ou organiques, caractérisés en ce que le pointillé représente une liaison carbonecarbone.

4) Les dérivés selon la revendication 1, 2 ou 3 ainsi que leurs sels d'addition avec les acides minéraux ou organiques caractérisés en ce que Ar représente un radical phényle éventuellement substitué.

5) Les dérivés selon la revendication 1, 2, 3 ou 4, ainsi que leurs sels d'addition avec les acides minéraux ou organiques, caractérisés en ce que $R_1$ et $R_2$ représentent un atome d'hydrogène.

6) Le 4-(6-méthoxy 1H-indol-3-yl) α-//2-(prop-2-ényloxy)phénoxy/ méthyl/pipéridine-1-éthanol, ainsi que ses sels d'addition avec les acides minéraux ou organiques.

7) Le 4-(1H-indol-3-yl) α-//2-(prop-2-ényloxy)phényloxy/ méthyl/1,2,3,6-tétrahydro pyridine-1-éthanol ainsi que ses sels d'addition avec les acides minéraux ou organiques.

8) Procédé de préparation des nouveaux dérivés du 4-(1H-indol-3-yl) α-méthyl pipéridine-1-éthanol, tels que définis par la formule (I) de la revendication 1 ainsi que de leurs sels d'addition avec les acides minéraux ou organiques caractérisé en ce que l'on fait réagir un dérivé de l'indole de formule (II):

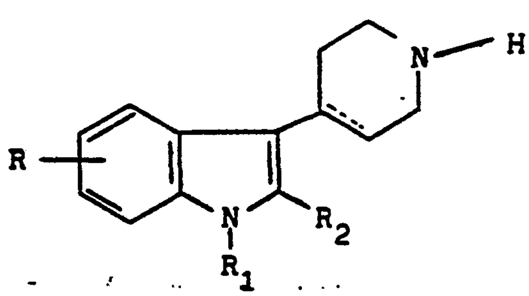

(II)

dans laquelle R, $R_1$ et $R_2$ ont la signification déjà indiquée, avec un époxyde de formule (III):

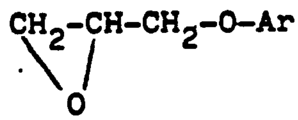

(III)

dans laquelle Ar a la signification déjà indiquée, pour obtenir un produit de formule (I) que l'on isole et si désiré salifie ou que l'on soumet si nécessaire ou si désiré à une ou plusieurs réactions suivantes dans un ordre approprié:

- déprotection du ou des groupements hydroxy protégés;
- réduction du ou des groupements nitro en groupements amino;
- acylation du ou des groupements amino;
- salification par un acide minéral ou organique.

9) Procédé selon la revendication 8, caractérisé en ce que:

- la réaction de l'indole de formule (II) avec l'époxyde de formule (III) est réalisée dans un mélange méthanol-solvant benzénique au reflux;
- lorsque le groupement Ar est substitué par un ou plusieurs groupements hydroxy, ceux-ci sont protégés sous forme de dérivés benzylés,
- la déprotection du ou des groupements hydroxy est effectué par réduction à l'hydrogène en présence d'un catalyseur tel que le palladium;
- la réduction du ou des groupements nitro est effectuée par l'hydrogène en présence d'un catalyseur tel que le palladium;
- l'acylation du ou des groupements amino est effectuée à l'aide d'un dérivé fonctionnel d'un acyle tel qu'un chlorure qu'un anhydride.

10) Médicaments, caractérisés en ce qu'ils sont constitués par les nouveaux dérivés du 4-(1H-indol-3-yl) α-méthyl pipéridine-1-éthanol, tels que définis par la formule (I) de la revendication 1 ainsi que par leurs sels d'addition avec les acides pharmaceutiquement acceptables.

11) Médicaments, caractérisés en ce qu'ils sont constitués par les nouveaux dérivés du 4-(1H-indol-3-yl) α-méthyl pipèridine-1-éthanol tels que dèfinis dans la revendication 2 ainsi que par leurs sels d'addition avec les acides pharmaceutiquement acceptables.

12) Médicaments, caractérisés en ce qu'ils sont constitués par les dérivés du 4-(1H-indol-3-yl) α-méthyl pipéridine-1-éthanol, tels que définis dans l'une des revendications 3, 4 ou 5, ainsi que par leurs sels d'addition avec les acides pharmaceutiquement acceptables.

13) Médicaments, caractérisès en ce qu'ils sont constitués par les dérivés du 4-(1H-indol-3-yl) α-méthyl pipéridine-1-éthanol tels que définis dans l'une des revendications 6 ou 7 ainsi que par leurs sels d'addition avec les acides pharmaceutiquement acceptables.

14) Compositions pharmaceutiques, caractérisées en ce qu'elles renferment, à titre de principe actif, l'un au moins des médicaments, tels que définis à l'une des revendications 10, 11, 12 ou 13.

**Revendications** pour l'Etat contractant AT

1) Procédé de préparation des dérivés du 4-(1H-indol-3-yl) α-méthyl pipéridine-1-éthanol, ainsi que de leurs sels d'addition avec les acides minéraux et organiques, répondant à la formule générale (I):

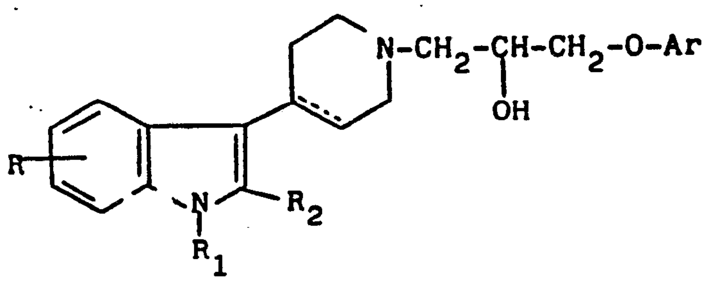

(I)

dans laquelle R représente un atome d'hydrogène ou d'halogène, ou un radical alkyle ou alkoxy renfermant de 1 à 5 atomes de carbone, nitro, amino, trifluorométhyle ou méthylthio, $R_1$ réprésente un atome d'hydrogène

ou un radical alkyle renfermant de 1 à 5 atomes de carbone, $R_2$ représente un atome d'hydrogène, un radical alkyle renfermant de 1 à 5 atomes de carbone ou un radical hydroxy, Ar représente un radical aryle ou hétéroaryle renfermant de 4 à 14 atomes de carbone éventuellement substitué par un ou plusieurs atomes de chlore, brome ou fluor, radicaux hydroxy, hydroxyméthyle, hydroxybutyle, méthoxy, éthoxy, propoxy, méthoxy-éthyle, carbamoyle, propényle, propényloxy, acétamido, butyrylamino, acétyle, nitro, amino, méthyle, éthyle, propyle, méthyl-sulfonamido, cyano, méthoxy-carbonyle, cyclohexyle, propynyle, éthynyle, propynyloxy ou trifluorométhyle et le pointillé représente la présence éventuelle d'une liaison carbone-carbone, caractérisé en ce que l'on fait réagir un dérivé de l'indole de formule (II):

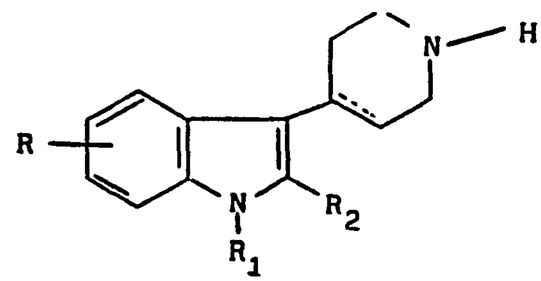

(II)

dans laquelle R, $R_1$ et $R_2$ ont la signification déjà indiquée, avec un époxyde de formule (III):

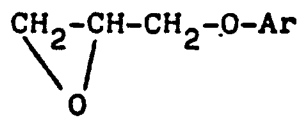

(III)

dans laquelle Ar a la signification déjà indiquée, pour obtenir un produit de formule (I) que l'on isole et si désiré salifie ou que l'on soumet si nécessaire ou si désiré à une ou plusieurs réactions suivantes dans un ordre approprié:

- déprotection du ou des groupements hydroxy protégés;
- réduction du ou des groupements nitro en groupements amino;
- acylation du ou des groupements amino;
- salification par un acide minéral ou organique.

2) Procédé selon la revendication 1, caractérisé en ce que:

- la réaction de l'indole de formule (II) avec l'époxyde de formule (III) est réalisée dans un mélange méthanol-solvant benzénique au reflux;
- lorsque le groupement Ar est substitué par un ou plusieurs groupements hydroxy, ceux-ci sont protégés sous forme de dérivés benzylés,
- la déprotection du ou des groupements hydroxy est effectuée par réduction à l'hydrogène en présence d'un catalyseur tel que le palladium;
- la réduction du ou des groupements nitro est effectuée par l'hydrogène en présence d'un catalyseur tel que le palladium;
- l'acylation du ou des groupements amino est effectuée à l'aide d'un dérivé fonctionnel d'un acyle tel qu'un chlorure ou un anhydride.

3) Procédé selon la revendication 1, pour préparer les dérivés du 4-(1H-indol-3-yl) α-méthyl pipéridine-1-éthanol, ainsi que de leurs sels d'addition avec les acides minéraux et organiques, répondant à la formule générale (I) dans laquelle R, $R_1$, Ar et le pointillé sont définis comme à la revendication 1 et $R_2$ représente un atome d'hydrogène ou un radical alkyle renfermant de 1 à 5 atomes de carbone, caractérisé en ce que l'on fait réagir un dérivé de l'indole de formule (II):

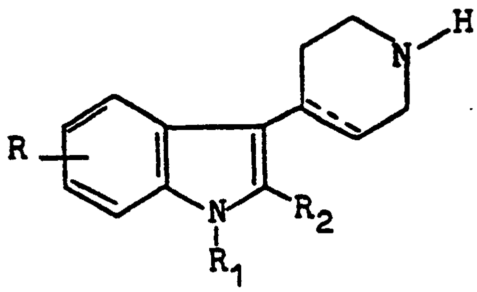

(II)

dans laquelle R, $R_1$ et $R_2$ ont la signification déjà indiquée, avec un époxyde de formule (III)

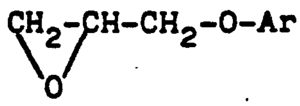

(III)

dans laquelle Ar a la signification déjà indiquée, pour obtenir un produit de formule (I) quel'on isole et, si désiré, salifie.

4) Procédé selon la revendication 3, caractérisé en ce que l'on utilise au départ, un produit de formule (II) dans laquelle le pointillé représente une liaison carbone-carbone.

5) Procédé selon la revendication 3 ou 4, caractérisé en ce que l'on utilise au départ un produit de formule (III) dans laquelle Ar représente un radical phényle éventuellement substitué.

6) Procédé selon la revendication 3, 4 ou 5, caractérisé en ce que l'on utilise au départ un produit de formule (II) dans laquelle $R_1$ et $R_2$ représente un atome d'hydrogène.

7) Procédé selon l'une quelconque des revendications 3 à 6, caractérisé en ce que les produits de formules II et III sont choisis de manière telle que l'on prépare le 4-(6-méthoxy 1H-indol-3-yl) α-//2-(prop-2-ényloxy)phénoxy/méthyl/ pipéridine-1-éthanol, ainsi que ses sels d'addition avec les acides minéraux ou organiques.

8) Procédé selon l'une quelconque des revendications 3 à 6, caractérisé en ce que les produits de formules II et III sont choisis de manière telle que l'on prépare le 4 -(1H-indol-3-yl) α-//2-(prop-2-ényloxy)phényloxy/méthyl/1,2,3,6-tétrahydro-pyridine-1-éthanol, ainsi que ses sels d'addition avec les acides minéraux ou organiques.

**Patentansprüche** die Vertragsstaaten: BE, CH, DE, FR, GB, IT, LI,

LU, NL, SE

1. 4-(1H-Indol-3-yl)-α-methylpiperidin-1-ethanol-Derivate sowie deren Additionssalze mit Mineral- und organischen Säuren, dadurch gekennzeichnet, daß sie der allgemeinen Formel (I)

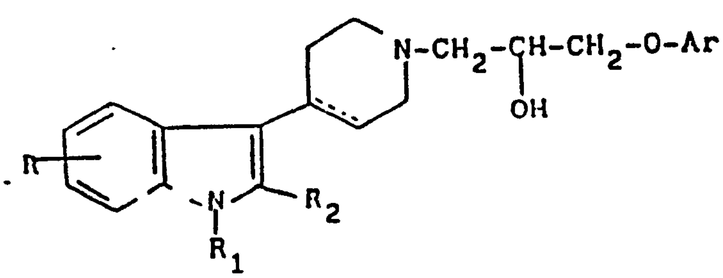

(I)

entsprechen, worin R ein Wasserstoff- oder Halogenatom oder einen Alkyl- oder Alkoxyrest mit 1 bis 3 Kohlenstoffatomen, Nitro, Amino, Trifluormethyl oder Methylthio bedeutet, $R_1$ ein Wasserstoffatom oder einen Alkylrest mit 1 bis 3 Kohlenstoffatomen darstellt, $R_2$ ein Wasserstoffatom, einen Alkylrest mit 1 bis 3 Kohlenstoffatomen oder einen Hydroxyrest bedeutet, Ar einen Aryl- oder Heteroarylrest mit 4 bis 14 Kohlenstoffatomen, gegebenenfalls substituiert durch ein oder mehrere Chlor-, Brom- oder Fluoratome, Hydroxy-, Hydroxymethyl-, Hydroxybutyl-, Methoxy-, Ethoxy-, Propoxy-, Methoxyethyl-, Carbamoyl-, Propenyl-, Propenyloxy-, Acetamido-, Butyrylamino-, Acetyl-, Nitro-, Amino-, Methyl-, Ethyl-, Propyl-, Methylsulfonamido-, Cyano-, Methoxycarbonyl-, Cyclohexyl-, Propinyl-, Ethinyl-, Propinyloxy- oder Trifluormethylreste bedeutet und die gestrichelte Linie die etwaige Anwesenheit einer Kohlenstoff-Kohlenstoff-Eindung anzeigt.

2. 4-(1H-Indol-3-yl)-α-methylpiperidin-1-ethanol-Derivate der Formel (I) gemäß Anspruch 1 sowie deren Additionssalze mit Mineral- oder organischen Säuren, dadurch gekennzeichnet daß $R_2$ ein Wasserstoffatom oder einen Alkylrest,mit 1 bis 3 Kohlenstoffatomen bedeutet.

3. Derivate gemäß Anspruch 1 oder 2 sowie deren Additionssalze mit Mineral- oder organischen Säuren, dadurch gekennzeichnet, daß die gestrichelte Linie eine Kohlenstoff-Kohlenstoff-Bindung anzeigt.

4. Derivate gemäß Anspruch 1, 2 oder 3 sowie deren Additionssalze mit Mineral- oder organischen Säuren, dadurch gekennzeichnet, daß Ar einen gegebenenfalls substituierten Phenylrest bedeutet.

5. Derivate gemäß Anspruch 1, 2, 3 oder 4 sowie deren Additionssalze mit Mineral- oder organischen Säuren, da durch gekennzeichnet, daß $R_1$ und $R_2$ ein Wasserstoffatom bedeuten.

6. 4-(6-Methoxy-1H-indol-3-yl)-α-[[2-(prop-2-enyl-oxy)-phenoxy]-methyl]-piperidin-1-ethanol sowie dessen Additionssalze mit Mineral- oder organischen Säuren.

7. 4-(1H-Indol-3-yl)-α-[[2-(prop-2-enyloxy)-phenyl-oxy]-methyl]-1,2,3,6-tetrahydro-pyridin-1-ethanol sowie dessen Additionssalze mit Mineral- oder organischen Säuren.

8. Verfahren zur Herstellung von neuen 4-(1H-Indol-3-yl)-α-methylpiperidin-1-ethanol-Derivaten der Formel (I) gemäß Anspruch 1 sowie von deren Additionssalzen mit Mineral- oder organischen Säuren, dadurch gekennzeichnet, daß man ein Indolderivat der Formel (II)

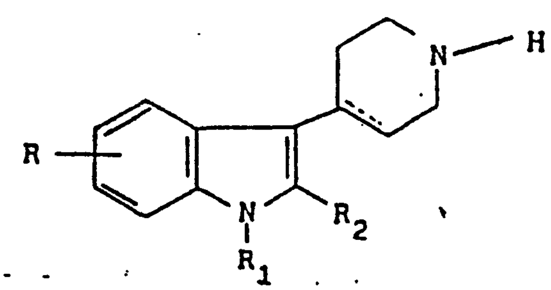

(II)

worin R, $R_1$ und $R_2$ die angegebene Bedeutung besitzen, mit,einen Epoxid der Formel (III)

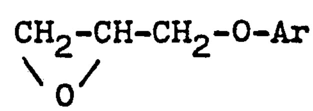

(III)

worin Ar die angegebene Bedeutung besitzt, umsetzt, um ein Produkt der Formel (I) zu erhalten, das man isoliert und gewünschtenfalls in ein Salz überführt, oder das man nötigenfalls oder gewünschtenfalls einer oder mehreren der folgenden Reaktionen in geeigneter Reihenfolge unterzieht:

Abspaltung der Schutzgruppe der geschützten Hydroxygruppe(n);
Reduktion der Nitrogruppe(n) in Aminogruppen;
Acylierung der Aminogruppe(n);
Salzbildung mit einer Mineral- oder organischen Säure.

9. Verfahren gemäß Anspruch 8, dadurch gekennzeichnet, daß

die Umsetzung des Indols der Formel (II) mit dem Epoxid der Formel (III) in einem Methanol-benzolischen Lösungsmittel-Gemisch unter Rückfluß erfolgt;

wenn die Gruppe Ar durch eine oder mehrere Hydroxygruppen substituiert ist, diese in Form der benzylierten Derivate geschützt sind;

die Abspaltung der Schutzgruppe der Hydroxygruppe(n) durch Reduktion mit Wasserstoff in Anwesenheit eines Katalysators, wie Palladium, durchgeführt wird;

die Reduktion der Nitrogruppe(n) durch Wasserstoff in Anwesenheit eines Katalysators, wie Palladium, durchgeführt wird;

die Acylierung der Aminogruppe(n) mit Hilfe eines funktionellen Acylderivats, wie eines Chlorids oder Anhydrids, durchgeführt wird.

10. Arzneimittel, dadurch gekennzeichnet, daß sie aus den neuen 4-(1H-Indol-3-yl)-α-methylpiperidin-1-ethanol-Derivaten der Formel (I) gemäß Anspruch 1 sowie aus deren Additionssalzen mit pharmazeutisch verträglichen Säuren bestehen.

11. Arzneimittel, dadurch gekennzeichnet, daß sie aus den neuen 4-(1H-Indol-3-yl)-α-methylpiperidin-1-ethanol-Derivaten, wie in Anspruch 2 definiert, sowie aus deren Additionssalzen mit pharmazeutisch verträglichen Säuren bestehen.

12. Arzneimittel, dadurch gekennzeichnet, daß sie aus den 4-(1H-Indol-3-yl)-α-methylpiperidin-1-ethanol-Derivaten gemäß einem der Ansprüche 3, 4 oder 3 sowie aus deren Additionssalzen mit pharmazeutisch verträglichen Säuren bestehen.

13. Arzneimittel, dadurch gekennzeichnet, daß sie aus den 4-(1H-Indol-3-yl)-α-methylpiperidin-1-ethanol-Derivaten gemäß einem der Ansprüche 6 oder 7 sowie aus deren Additionssalzen mit pharmazeutisch verträglichen Säuren bestehen.

14. Pharmazeutische Zusammensetzungen, dadurch gekennzeichnet, daß sie als Wirkstoff zumindest eines der Arzneimittel gemäß einem der Ansprüche 10, 11, 12 oder 13 enthalten.

**Patentansprüche** für den Vertragsstaat: AT

1. Verfahren zur Herstellung von 4-(1H-Indol-3-yl)-α-methylpiperidin-1-ethanol-Derivaten sowie von deren Additionssalzen mit Mineral- und organischen Säuren der allgemeinen Formel (I)

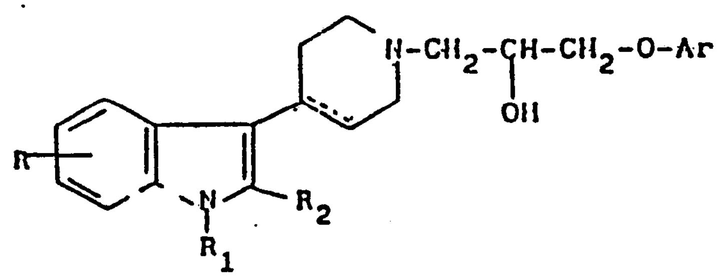

(I)

worin R ein Wasserstoff- oder Halogenatom oder einen Alkyl- oder Alkoxyrest mit 1 bis 3 Kohlenstoffatomen, Nitro, Amino, Trifluormethyl oder Methylthio bedeutet, $R_1$ ein Wasserstoffatom oder einen Alkylrest mit 1 bis 3 Kohlenstoffatomen darstellt, $R_2$ ein Wasserstoffatom, einen Alkylrest mit 1 bis 3 Kohlenstoffatomen oder einen Hydroxyrest bedeutet, Ar einen Aryl- oder Heteroarylrest mit 4 bis 14 Kohlenstoffatomen, gegebenenfalls substituiert durch ein oder mehrere Chlor-, Brom- oder Fluoratome, Hydroxy-, Hydroxymethyl-, Hydroxybutyl-, Methoxy-, Ethoxy-, Propoxy-, Methoxyethyl-, Carbamoyl-, Fropenyl-, Propenyloxy-, Acetamido-, Butyrylamino-, Acetyl-, Nitro-, Amino-Methyl-, Ethyl-, Propyl-, Methylsulfonamido-, Cyano-, Methoxycarbonyl-, Cyclohexyl-, Propinyl-, Ethinyl-, Propinyloxy- oder Trifluormethylreste bedeutet und die gestrichelte Linie die etwaige Anwesenheit einer Kohlenstoff-Kohlenstoff-Bindung anzeigt, dadurch gekennzeichnet, daß man ein Indolderivat der Formel (II)

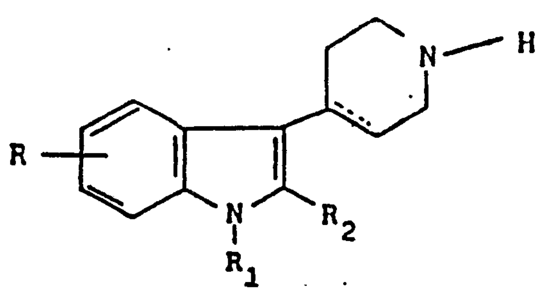

(II)

worin R, $R_1$ und $R_2$ die angegebene Bedeutung besitzen, mit einem Epoxid der Formel (III)

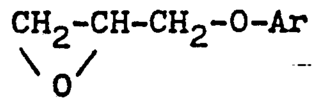
(III)

worin Ar die angegebene Bedeutung besitzt, umsetzt, um ein Produkt der Formel (I) zu erhalten, das man isoliert und gewünschtenfalls in ein Salz überführt, oder das man nötigenfalls oder gewünschtenfalls einer oder mehreren der folgenden Reaktionen in geeigneter Reihenfolge unterzieht:
Abspaltung der Schutzgruppe der geschützten Hydroxygruppe(n);
Reduktion der Nitrogruppe(n) in Aminogruppen;
Acylierung der Aminogruppe(n);
Salzbildung mit einer Mineral- oder organischen Säure.

2. Verfahren gemäß Anspruch 1, dadurch gekennzeichnet, daß
die Umsetzung des Indols der Formel (II) mit dem Epoxid der Formel (III) in einem Methanol-benzolischen Lösungsmittel-Gemisch unter Rückfluß erfolgt;
wenn die Gruppe Ar durch eine oder mehrere Hydroxygruppen substituiert ist, diese in Form der benzylierten Derivate geschützt sind;
die Abspaltung der Schutzgruppe der Hydroxygruppe(n) durch Reduktion mit Wasserstoff in Anwesenheit eines Katalysators, wie Falladium, durchgeführt wird;
die Reduktion der Nitrogruppe(n) mit Wasserstoff in Anwesenheit eines Katalysators, wie Palladium, erfolgt;
die Acylierung der Aminogruppe(n) mit Hilfe eines funktionellen Acylderivats, wie eines Chlorids oder Anhydrids, erfolgt.

3. Verfahren gemäß Anspruch 1 zur Herstellung der 4-(1H-Indol-3-yl)-α-methylpiperidin-1-ethanol-Derivate sowie von deren Additionssalzen mit Mineral- und organischen Säuren der allgemeinen Formel (I), worin R, $R_1$, Ar und die gestrichelte Linie wie in Anspruch 1 definiert sind und $R_2$ ein Wasserstoffatom oder einen Alkylrest mit 1 bis 3 Kohlenstoffatomen bedeutet, dadurch gekennzeichnet, daß man ein Indolderivat der Formel (II)

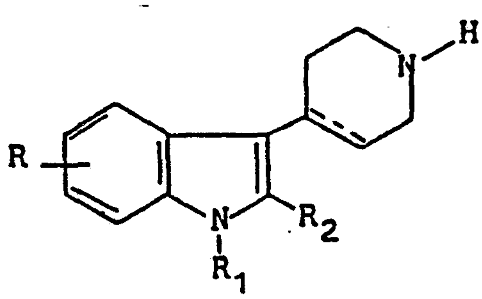
(II)

worin R, $R_1$ und $R_2$ die angegebene Bedeutung besitzen, mit einem Epoxid der Formel (III)

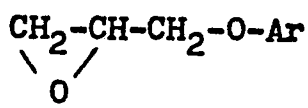
(III)

worin Ar die angegebene Bedeutung besitzt, umsetzt, um ein Produkt der Formel (I) zu erhalten, das man isoliert und gewünschtenfalls in ein Salz überführt.

4. Verfahren gemäß Anspruch 3, dadurch gekennzeichnet, daß man von einem Produkt der Formel (II) ausgeht, worin die gestrichelte Linie eine Kohlenstoff-Kohlenstoff-Bindung darstellt.

5. Verfahren gemäß Anspruch 3 oder 4, dadurch gekennzeichnet, daß man von einem Produkt der Formel (III) ausgeht, worin Ar einen gegebenenfalls substituierten Phenylrest darstellt.

6. Verfahren gemäß Anspruch 3, 4 oder 3, dadurch gekennzeichnet, daß man von einem Produkt der Formel (II) ausgeht, worin $R_1$ und $R_2$ ein Wasserstoffatom bedeuten.

7. Verfahren gemäß einem der Ansprüche 3 bis 6, dadurch gekennzeichnet, daß die Frodukte der Formeln II und III derart ausgewählt werden, daß man das 4-(6-Methoxy-1H-indol-3-yl)-α-[[2-(prop-2-enyloxy)-phenoxy]-me-thyl]-piperidin-1-ethanol sowie dessen Additionssalze mit Mineral- oder organischen Säuren herstellt.

8. Verfahren gemäß einem der Ansprüche 3 bis 6, dadurch gekennzeichnet, daß die Frodukte der Formeln II und III derart ausgewählt werden, daß man das 4-(1H-Indol-3-yl)-α-[[2-(prop-2-enyloxy)-phenyloxy]-methyl]-1,2,3,6-tetrahydropyridin-1-ethanol sowie dessen Additionssalze mit Mineral- oder organischen Säuren herstellt.

**Claims** for the contracting states BE CH DE FR CB IT LI LU NL SE

1) The derivatives of 4-(1H-indol-3-yl)α-methyl piperidine-1-ethanol, as well as their salts of addition with mineral or organic acids, characterised in that they answer to the general formula (I):

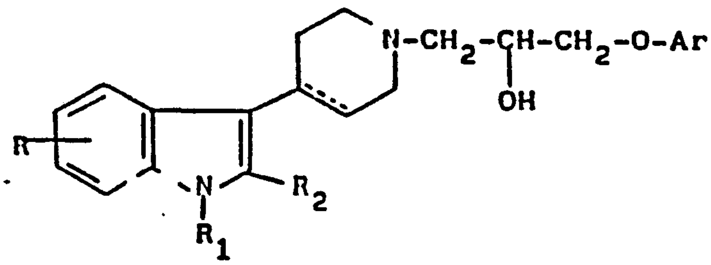

(I)

in which R represents a hydrogen or halogen atom, or an alkyl or alkoxy radical containing from 1 to 5 carbon, nitro, amino, trifluoromethyl or methylthio atoms, $R_1$ represents a hydrogen atom or an alkyl radical containing from 1 to 5 carbon atoms, $R_2$ represents a hydrogen atom, an alkyl radical containing from 1 to 5 carbon atoms or a hydroxy radical, Ar represents an aryl or hetero-aryl radical containing from 4 to 14 carbon atoms possibly substituted by one or more chlorine, bromine or fluorine atoms, or hydroxy, hydroxymethyl, hydroxybutyl, methoxy, ethoxy, propoxy, methoxy-ethyl, carbamoyl, propenyl, propenyloxy, acetamido, butyrylamino, acetyl, nitro, amino, methyl, ethyl, propyl, methylsulphonamido, cyano, methoxy-carbonyl, cyclohexyl, propynyl, ethynyl, propynyloxy or trifluoromethyl radicals, and the dotted line represents the possible presence of a carbon-carbon bond.

2) The derivatives of 4-(1H-indol-3-yl)α-methyl piperidine-1-ethanol, as defined by formula (I) of claim 1, as well as their salts of addition with mineral or organic acids, characterised in that $R_2$ represents a hydrogen atom or an alkyl radical containing from 1 to 5 carbon atoms.

3) The derivatives according to claim 1 or 2, as well as their salts of addition with mineral or organic acids, characterized in that the dotted line represents a carbon-carbon bond.

4) The derivatives according to claim 1, 2 or 3 as well as their salts of addition with mineral or organic acids, characterized in that Ar represents a possibly substituted phenyl radical.

5) The derivatives according to claim 1, 2, 3 or 4, as well as their salts of addition with mineral or organic acids, characterized in that $R_1$ and $R_2$ represent a hydrogen atom.

6) 4-(6-methoxy 1H-indol-3-yl)α-[[2-(prop-2-enyloxy)phenoxy]-methyl]piperidine-1-ethanol, as well as its salts of addition with mineral or organic acids.

7) 4-(1H-indol-3-yl)α-[[2-(prop-2-enyloxy)phenyloxy]methyl]-1,2,3,6-tetrahydro pyridine-1-ethanol as well as its salts of addition with mineral or organic acids.

8) Preparation process for the new derivatives of 4-(1H-indol-3-yl)α-methyl piperidine-1-ethanol, as defined by formula (I) of claim 1, as well as their salts of addition with mineral or organic acids characterized in that a derivative of indole with the formula (II):

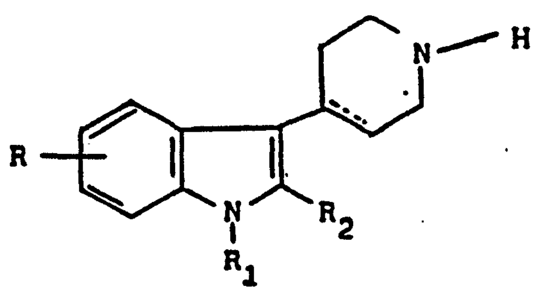

(II)

in which R, $R_1$ and $R_2$ have the significance already indicated, is made to react with an epoxide with the formula (III):

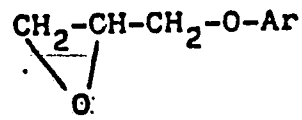

(III)

in which Ar has the significance already indicated, so as to obtain a product with the formula (I) which is isolated and, if desired, salified or is submitted, if necessary or desired, to one or more of the following reactions in an appropriate order:

- deprotection of the protected hydroxy group or groups;
- reduction of the nitro group or groups into amino groups;
- acylation of the amino group or groups;
- salification by a mineral or organic acid.

9) Process according to claim 8, characterized in that:

- the reaction of the indole with the formula (II) with the epoxide with the formula (III) is carried out in a benzenemethanol solvent mixture at reflux;
- when the Ar group is substituted by one or more hydroxy groups, the latter are protected in the form of benzyl derivatives;
- the deprotection of the hydroxy group or groups is carried out by reduction with hydrogen in the presence of a catalyst such as palladium;
- the reduction of the nitro group or groups is carried out by hydrogen in the presence of a catalyst such as palladium;
- the acylation of the amino group or groups is carried out by a functional derivative of an acyl such as a chloride or an anhydride.

10) Medicaments, characterized in that they are constituted by the new derivatives of 4-(1H-indol-3-yl)α-methyl piperidine-1-ethanol, as defined by formula (I) of claim 1, as well as by their salts of addition with pharmaceutically acceptable acids.

11) Medicaments, characterized in that they are constituted by the new derivatives of 4-(1H-indol-3-yl)α-methyl piperidine-1-ethanol as defined in claim 2, as well as by their salts of addition with pharmaceutically acceptable acids.

12) Medicaments, characterized in that they are constituted by the derivatives of 4-(1H-indol-3-yl)α-methyl piperidine-1-ethanol, as defined in any one of the claims 3, 4 or 5, as well as by their salts of addition with pharmaceutically acceptable acids.

13) Medicaments, characterized in that they are constituted by the derivatives of 4-(1H-indol-3-yl)α-methyl piperidine-1-ethanol as defined in either one of the claims 6 or 7, as well as their salts of addition with pharmaceutically acceptable acids.

14) Pharmaceutical compositions, characterized in that they contain, as active principle, one at least of the medicaments, as defined in any one of the claims 10, 11, 12 or 13.

**Claims** for the contracting state AT

1) Preparation process for the derivatives of 4-(1H-indol-3-yl)α -methyl piperidine-1-ethanol, as well as their salts of addition with mineral or organic acids, answering to the general formula (I):

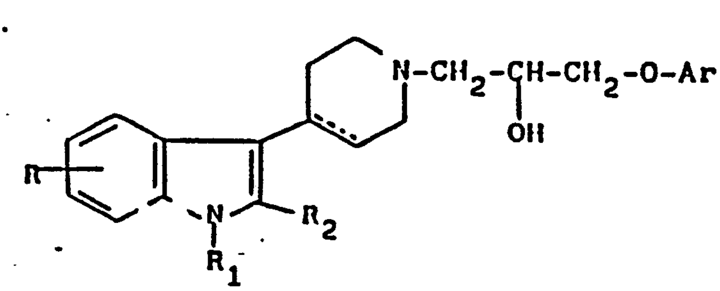

(I)

in which R represents a hydrogen or halogen atom, or an alkyl or alkoxy radical containing from 1 to 5 carbon, nitro, amino, trifluoromethyl or methylthio atoms, $R_1$ represents a hydrogen atom or an alkyl radical containing from 1 to 5 carbon atoms, $R_2$ represents a hydrogen atom, an alkyl radical containing from 1 to 5

carbon atoms or a hydroxy radical, Ar represents an aryl or hetero-aryl radical containing from 4 to 14 carbon atoms possibly substituted by one or more chlorine, bromine or fluorine atoms, or hydroxy, hydroxymethyl, hydroxybutyl, methoxy, ethoxy, propoxy, methoxy-ethyl, carbamoyl, propenyl, propenyloxy, acetamido, butyrylamino, acetyl, nitro, amino, methyl, ethyl, propyl, methylsulphonamido, cyano, methoxy-carbonyl, cyclohexyl, propynyl, ethynyl, propynyloxy or trifluoromethyl radicals, and the dotted line represents the possible presence of a carbon-carbon bond, characterized in that a derivative of indole with the formula (II):

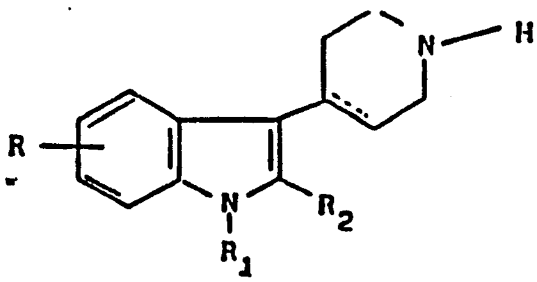

(II)

in which R, $R_1$ and $R_2$ have the significance already indicated, is made to react with an epoxide with the formula (III):

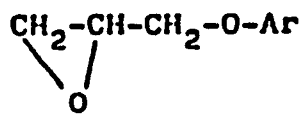

(III)

in which Ar has the significance already indicated, so as to obtain a product with the formula (I) which is isolated and, if desired, salified, or which is submitted, if necessary or desired, to one or more of the following reactions in an appropriate order:
- deprotection of the protected hydroxy group or groups;
- reduction of the nitro group or groups into amino groups;
- acylation of the amino group or groups;
- salification by a mineral or organic acid.

2) Process according to claim 1, characterized in that:
- the reaction of the indole with the formula (II) with the epoxide with the formula (III) is carried out in a benzenemethanol solvent mixture at reflux;
- when the Ar group is substituted by one or more hydroxy groups, the latter are protected in the form of benzyl derivatives;
- the deprotection of the hydroxy group or groups is carried out by reduction with hydrogen in the presence of a catalyst such as palladium;
- the reduction of the nitro group or groups is carried out by hydrogen in the presence of a catalyst such as palladium;
- the acylation of the amino group or groups is carried out by a functional derivative of an acyl such as chloride or anhydride.

3) Process according to claim 1, for preparing derivatives of 4-(1H-indol-3-yl)α-methyl piperidine-1-ethanol, as well as their salts of addition with mineral or organic acids, answering to the general formula (I) in which R, $R_1$, Ar and the dotted line are defined as in claim 1 and $R_2$ represents a hydrogen atom or an alkyl radical containing from 1 to 5 carbon atoms, characterized in that a derivative of indole with the formula (II):

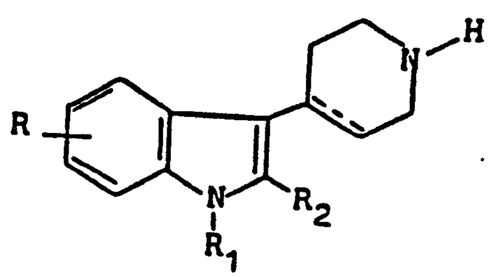

(II)

in which R, $R_1$ and $R_2$ have the significance already indicated, is made to react with an epoxide with the formula (III):

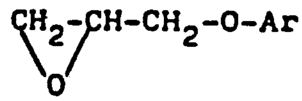

(III)

in which Ar has the significance already indicated, so as to obtain a product with the formula (I) which is isolated and, if desired, salified

4) Process according to claim 3, characterized in that at the beginning a product with the formula (II), in which the dotted line represents a carbon-carbon bond, is used.

5) Process according to claim 3 or 4, characterized in that at the beginning a product with the formula (III), in which Ar represents a possibly substituted phenyl radical, is used.

6) Process according to claim 3, 4 or 5, characterized in that at the beginning a product with the formula (II), in which $R_1$ and $R_2$ represent a hydrogen atom, is used.

7) Process according to any one of the claims 3 to 6, characterized in that the products with the formulae (II) and (III) are chosen in such a way that 4-(6-methoxy-1H-indol-3-yl)α-[[2-(prop-2-enyloxy)phenoxy]methyl]piperidine-1-ethanol is prepared, as well as its salts of addition with mineral or organic acids.

8) Process according to any one of the claims 3 to 6, characterized in that the products with the formulae (II) and (III) are chosen in such a way that 4-(1H-indol-3-yl)α-[[2-(prop-2-enyloxy)-phenyloxy]methyl] 1,2,3,6-tetrahydropyridine-1-ethanol is prepared, as well as its salts of addition with mineral or organic acids.